# EUROPEAN PATENT APPLICATION

(11) **EP 1 990 372 A2**
(43) Date of publication of application: **12.11.2008**
(21) Application number: 07107820.8
(22) Date of filing: 09.05.2007
(51) Int. Cl.: C09B 5/62

(54) **Water soluble UV filters**

(71) Applicant: Ciba Holding Inc., 4057 Basel (CH)
(72) Inventor: Wagner, Barbara., 79539 Lörrach (DE)

(57) **Abstract**

Disclosed is the use of the compounds of formula 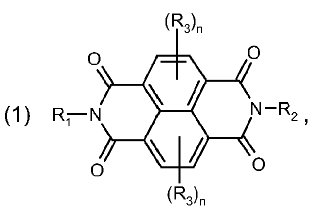 wherein 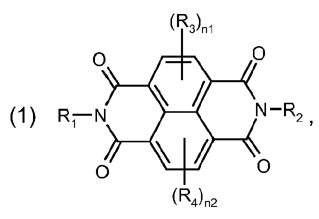 wherein
R₁ and R₂ independently of each other are hydrogen; C₁-C₂₈alkyl, C₂-C₂₈alkenyl, C₂-C₂₂alkinyl, C₃-C₁₂cycloalkyl, C₃-C₁₂cycloalkenyl, C₇-C₂₈aralkyl, C₁-C₂₀heteroalkyl, C₃-C₁₂cycloheteroalkyl, C₅-C₁₆heteroaralkyl, which are substituted by one or more groups of Z and/or interrupted by -O-; -NR₆-; or CO-R₅;
with the proviso that R₁ and R₂ are not simultaneously hydrogen;
Z is COO(M)ₘ *-SO₃(M)ₘ; or *-SO₂NR₆R₇;
M is hydrogen; or a cation;
m is 1; 0.5; or ⅓;
R₃ and R₄, independently from each other are C₁-C₂₈alkyl; C₂-C₂₈ alkenyl; C₂-C₂₈alkinyl; C₃-C₁₂cydoalkyl; C₃-C₁₂cycloalkenyl; C₇-C₂₀aralkyl; C₂-C₂₈alkoxy; or OH;
R₅, R₆ and R₇ independently from each other are hydrogen; C₁-C₂₈alkyl; hydroxyl-C₁-C₂₈alkyl; C₂-C₂₈alkenyl; C₂-C₂₈alkinyl; C₃-C₁₂cycloalkyl; C₃-C₁₂cycloalkenyl; C₇-C₂₀aralkyl; C₂-C₂₈ alkoxy; or OH; and
n1 and n2, independently from each other are0; 1; or 2;
for protecting human and animal hair and skin from UV radiation.

The compounds of formula (1) represent useful watersoluble broad-band UV filters which cover the whole UV light range from about 290 to 380 nm.

## Description

The present invention relates to the use of selected naphthalenebisimide derivatives for protecting human and animal hair and skin from harmful effects of UV radiation.

### Background of the invention

It is well known that ultraviolet radiation (light) is harmful to human skin. Depending on the wavelength the UV radiation causes different types of skin damage. UV-B radiation (about 290 to about 320 nm) is responsible for sunburn and can cause skin cancer. UV-A radiation (about 320 to about 400 nm) while producing tanning of the skin, contributes also to sunburn and the induction of skin cancers. Moreover, the harmful effects of the UV-B radiation may be aggravated by UV-A radiation.

The effects of UV-A are mainly mediated by free radicals, e.g. reactive oxygen species inducing different types of degradation to cellular DNA, lipids, and proteins. The visible signs are often the result of long-term, cumulative effects. This is why skin photoaging is associated with UV-A light. It is also known that normal outdoor UV-A radiation can be effective enough to cause the breakdown of the proteins collagen and elastin leading to a loss of firmness and resilience of the skin. Therefore the UVA protection of a daily skin care is of significant relevance.

Therefore, an effective sunscreen formulation preferably comprises both at least one UV-A and UV-B filter covering the full range of about 290 nm to about 400 nm to prevent the human skin from damaging by the sunlight. The solubility of the UV filter substances in the oil- and water-phases is of crucial importance for a dermatological formulation, because for the adjustment of a high sun protection factor (also called SPF) it is necessary to incorporate UV filters in all phases of a formulation. Therefore it is desirable to have oil- as well as watersoluble UV filters.

Furthermore UV filters are differentiated between UV-B filters (absorption between 280 and 320 nm), UV-A filters (absorption between 320 and 400 nm) and broad band filters (absorption between 290 and approximately 380 nm).

Numerous UV-B filters are registered for their use in sunscreen preparations, which are mainly derivatives of the 3-benzylidenecampher, ethylhexyl salicylates and p-methoxycinnamic acid esters, such as 2-ethylhexyl p-methoxycinnamate. A commercial UV-B filter which is applicable in the water phase of a cosmetic formulation is 2-phenyl-1 H-benzimidazole-5-sulfonic acid (INCI: phenyl benzimidazole sulfonic acid).

The most commonly used commercial UV-A filter is a dibenzoylmethane derivative, particularly the 4-(tert-butyl)-4'-methoxydibenzoylmethane (also called avobenzone, CAS No. 70356-09-1), which is commercialised by DSM under the brand name Parsol® 1789 and Merck under the brand name Eusolex® 9020. Other dibenzoylmethane derivatives are described in U.S. Pa. Nos. 4,489,057, 4,387,089 and 4,562,067. UV-A filters comprising the dibenzoylmethane structure are used in the oil phase of a personal care formulation.

Watersoluble UV-A filters are derivatives of the phenylenedimethylene structure like the commercial filter 3,3'-(1,4-phenylenedimethylene)bis[7,7-dimethyl-2-oxo-bicyclo[2.2.1]hep-tate-1-methanesulfonic acid (INCI: terephthalylidene dicamphor sulfonic acid) or of the dibenzimidazole structure like the commercial filter 2,2'-(1,4-phenylene)bis-[1H-benzimidazole-4,6-disulfonic acid] disodium salt (INCI: disodium phenyl dibenzimidazole tetrasulfonate).

The so-called broad-band filters cover the UV-B as well as the UV-A region. Commonly used broad band filters are the unsymmetrically substituted s-triazine-compound 2,4-bis-{[4-(2-ethyl-hexyloxy)-2-hydroxy]-phenyl}-6-(4-methoxyphenyl)-1,3,5-triazine (INCI: bis-ethylhexyloxyphenol methoxyphenyl triazine) which is commercialized by Ciba Specialty Chemicals under the brand name Tinosorb S® and particular benzophenone derivatives like 2-hydroxy-4-methoxy-benzophenone (INCI: benzophenone-3). Both commercial broad band filters are applied in the oil phase of a dermatological formulation. Another commercial broad band filter is the particulate UV filter 2,2'-methylene-bis-{6-(2H-benzotriazole-2-yl)-4-(1,1,3,3-tetramethylbutyl)-phenol) (INCI: methylene bis-benzotriazolyl tetramethyl butylphenol}.

While there are commercial watersoluble UV-B filters and UV-A filters known there is still need for watersoluble broad-band UV filters which cover the whole UV light range from about 290 to 380 nm.

One challenge of this invention was therefore to provide watersoluble UV absorbers with absorbance properties in particular in the UV-A but also in the UV-B region.

It was found that specific watersoluble naphthalene bisimide derivatives can solve this task.

Therefore, the present invention relates to the use of the compounds of formula 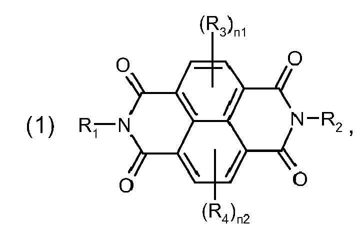 wherein
- R₁ and R₂: independently of each other are hydrogen; C₁-C₂₈alkyl, C₂-C₂₈alkenyl, C₂-C₂₂alkinyl, C₃-C₁₂cycloalkyl, C₃-C₁₂cycloalkenyl, C₇-C₂₈aralkyl, C₁-C₂₀heteroalkyl, C₃-C₁₂cycloheteroalkyl, C₅-C₁₆heteroaralkyl, which are substituted by one or more groups of Z and/or interrupted by -O-; -NR₆-; or CO-R₅;
with the proviso that R₁ and R₂ are not simultaneously hydrogen;
- Z: is COO(M)ₘ *-SO₃(M)ₘ; or -SO₂NR₆R₇;
- M: is hydrogen; or a cation;
- m: is 1; 0.5; or ¹/₃;
- R₃ and R₄,: independently from each other are C₁-C₂₈alkyl; C₂-C₂₈ alkenyl; C₂-C₂₈alkinyl; C₃-C₁₂cycloalkyl; C₃-C₁₂cycloalkenyl; C₇-C₂₀aralkyl; C₂-C₂₈alkoxy; or OH;
- R₅, R₆ and R₇: independently from each other are hydrogen; C₁-C₂₈alkyl; hydroxyl-C₁-C₂₈alkyl; C₂-C₂₈alkenyl; C₂-C₂₈alkinyl; C₃-C₁₂cycloalkyl; C₃-C₁₂cycloalkenyl; C₇-C₂₀aralkyl; C₂-C₂₈ alkoxy; or OH; and
- n 1 and n2,: independently from each other are 0; 1; or 2;
for protecting human and animal hair and skin from UV radiation.

Alkyl, cycloalkyl, alkenyl, alkylidene or cycloalkenyl residues can be straight-chain or branched, monocyclic or polycyclic. Alkyl can be for example methyl, straight-chain C₂-C₂₈ alkyl or preferably branched C₃-C₁₂ alkyl. Alkenyl can be e.g. straight-chain C₂-C₂₈ alkenyl or preferably branched C₃-C₁₂alkenyl. C₁-C₁₂Alkyl is for example methyl, ethyl, n-propyl, isopropyl, n-butyl, sec.-butyl, isobutyl, tert.-butyl, n-pentyl, 2-pentyl, 3-pentyl, 2,2-dimethylpropyl, n-hexyl, n-octyl, 1,1,3,3-tetramethylbutyl, 2-ethylhexyl, nonyl, decyl, n-octadecyl, eicosyl, oder dodecyl. C₃-C₁₂Cycloalkyl is for example cyclopropyl, cyclobutyl, cyclopentyl, cyclohexyl, trimethylcyclohexyl, menthyl, thujyl, bornyl, 1-adamantyl oder 2-adamantyl.

C₂-C₂₈ alkenyl or C₃-C₁₂ cycloalkenyl refers to unsaturated hydrocarbon residues containing one or multiple double bonds such vinyl, allyl, 2-propen-2-yl, 2-buten-1-yl, 3-buten-1-yl, 1,3-butadien-2-yl, 2-cyclobuten-1-yl, 2-penten-1-yl, 3-penten-2-yl, 2-methyl-1-buten-3-yl, 2-methyl-3-buten-2-yl, 3-methyl-2-buten-1-yl, 1,4-pentadien-3-yl, 2-cyclopenten-1-yl, 2-cyclohexen-1-yl, 3-cyclohexen-1-yl, 2,4-cyclohexadien-1-yl, 1-*p*-menthen-8-yl, 4(10)-thujen-10-yl, 2-norbornen-1-yl, 2,5-norbornadien-1-yl, 7,7-dimethyl-2,4-norcaradien-3-yl or signifies different isomers of hexenyl, octenyl, nonenyl, decenyl or dodecenyl.

C₇-C₂₈aralkyl is for example benzyl, 2-benzyl-2-propyl, β-phenyl-ethyl, 9-fluorenyl, α,α-dimethylbenzyl, ω-phenyl-butyl, ω-phenyl-octyl, ω-phenyl-dodecyl oder 3-methyl-5-(1',1',3',3'-tetramethyl-butyl)-benzyl. A C₇-C₂₈ aralkyl moiety can be unsubstituted or substituted at the alkyl- as well at the aryl-moiety of the aralkyl-group, but preferably substituted at the aryl-moiety.

C₅-C₁₆heteroaralkyl is for example a C₁-C₈ alkyl moiety which is substituted with a C₄-C₈heteroaryl group.

M denotes a cation like for example a metal cation like sodium, potassium, calcium, magnesium, or lithium; or may denote an organic cation like ammonium, triethanolammonium or the like;

Preferred is the use of compounds of formula (1), wherein
- R₁: and R₂ independently of each other are hydrogen; or C₁-C₂₈alkyl, which are substituted by one or more groups of Z and/or interrupted by -O- or -NR₆-; wherein
- Z and R₆: are defined as in formula (1.)

More preferred are compounds of formula (1), wherein
- R₁ and R₂: independently of each other are C₁-C₅alkyl.

Even more preferred are compounds of formula (1), wherein
- R₁ and R₂: have the same meaning.

Preferred is the use of compounds of formula (1), wherein
- M: is a metal cation selected from sodium, potassium, calcium, magnesium and lithium; or an organic cation selected from ammonium and triethanolammonium.

Preferred are also compounds of formula (1), wherein
- n1 and n2: are 0.

Most preferred are compounds of formula (1), wherein
- Z: is *-SO₃H; *-SO₂NH₂ or *-COOM; wherein
- M: is hydrogen; or an alkali metal cation.

Most preferred compounds correspond to formula 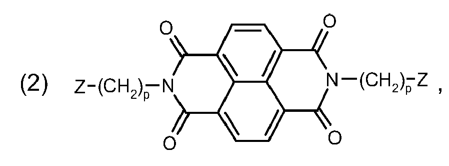 wherein
- Z: is *-SO₃H; *-SO₂NH₂ or *-COOM;
- M: is hydrogen; or an alkali metal cation; and
- p: is a number from 1 to 5; and
wherein the (CH₂)-chains may be substituted by hydroxyl, -NR₆R₇ and/or interrupted by -O-; and
- R₆ and R₇: are defined as in formula (1).

In a further aspect, the invention also concerns a cosmetic or dermatological composition for topical use, characterized in that it comprises at least one UV screen of the watersoluble naphthalene derivative type in a cosmetically acceptable support.

Examples of watersoluble naphthalene bisimide derivatives of the present invention which are listed in Table 1:

| Table 1: Examples of naphthalene bisimide derivatives according to the present invention | | |
|---|---|---|
| Comp. Nr. | Formula | λₘₐₓ |
| NB-01 | 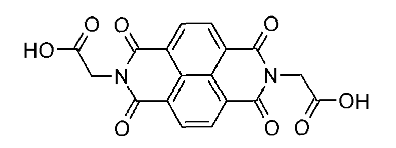 | |
| NB-02 | 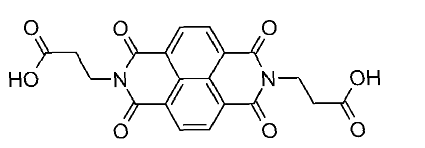 | |
| NB-03 | 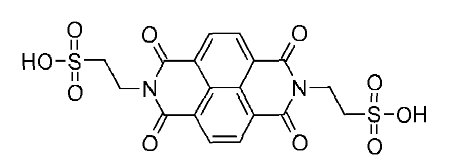 | |
| NB-04 | 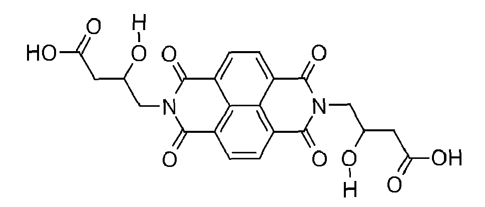 | |
| NB-05 | 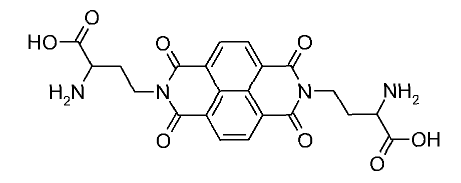 | |
| NB-06 | 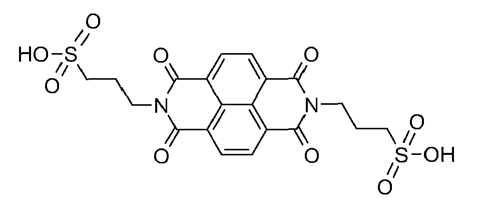 | |
| NB-07 | 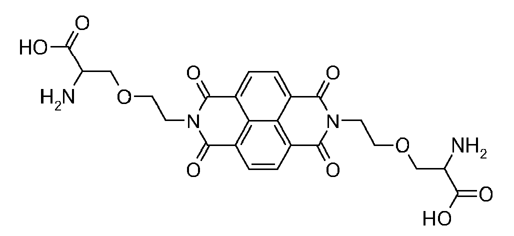 | |
| NB-08 | 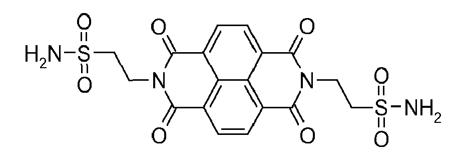 | |
| NB-09 | 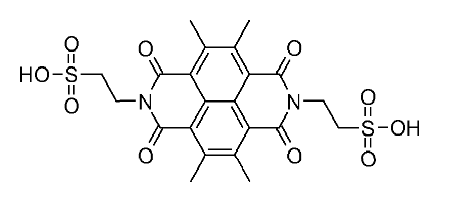 | |
| NB-10 | 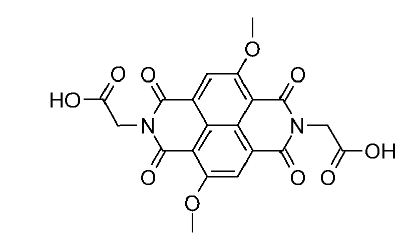 | |
| NB-11 | 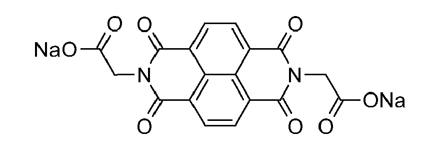 | |
| NB-12 | 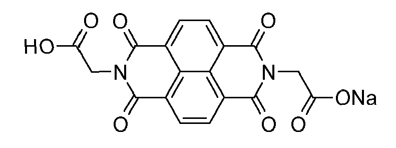 | |
| NB-13 | 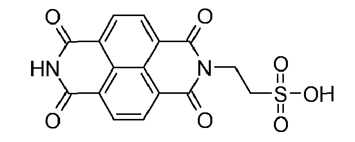 | |
| NB-14 | 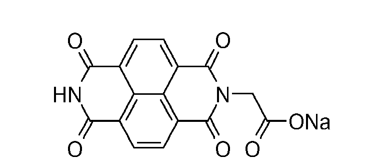 | |

Naphthalene bisimide derivatives of formula 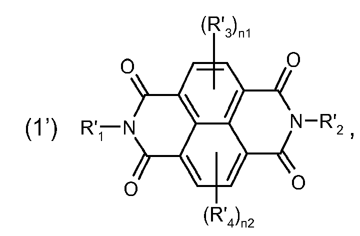 wherein
- R'₁ and R'₂: independently of each other are hydrogen; C₁-C₂₈alkyl, C₂-C₂₈alkenyl, C₂-C₂₂alkinyl, C₃-C₁₂cycloalkyl, C₃-C₁₂cycloalkenyl, C₇-C₂₈aralkyl, C₁-C₂₀heteroalkyl, C₃-C₁₂cycloheteroalkyl, C₅-C₁₆heteroaralkyl, which are substituted by one or more groups of Z' and/or interrupted by -O- or -NR'₆-;
with the proviso that R'₁ and R'₂ are not simultaneously hydrogen;
- Z': is *-SO₃(M')ₘ; or *-SO₂NR'₆R'7₈;
- M': is hydrogen; or a cation;
- m: is 1; 0.5; or ¹/₃;
- R'₃ and R'₄: independently from each other are C₁-C₂₈alkyl; C₂-C₂₈ alkenyl; C₂-C₂₈alkinyl; C₃-C₁₂cycloalkyl; C₃-C₁₂cycloalkenyl; C₇-C₂₀aralkyl; C₂-C₂₈alkoxy; or OH;
- R'₅, R'₆ and R'₇: independently from each other are hydrogen; C₁-C₅alkyl; or hydroxy-C₁-C₅alkyl; and
- n1 and n2: independently from each other are 0; 1; or 2;
are novel and represent a further embodiment of the present invention.

Naphthalene bisimide derivatives of formula (1) wherein R₁ = R₂ may be prepared using a method as described in the journal "Chemistry - a European Journal" (Chem.Eur.J.) in the year 2006 (volume 12) on the pages 2815-2824 in accordance with the following reaction scheme:

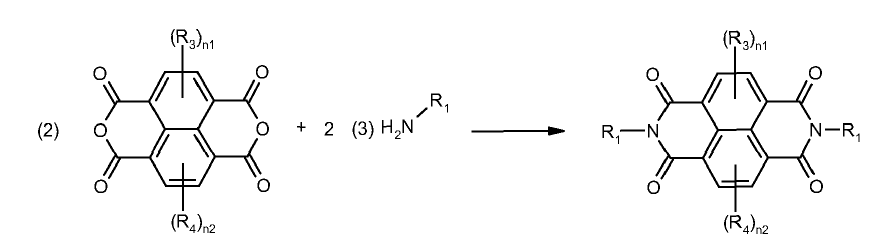

Naphthalene bisimide derivatives of formula (1) wherein R₁ ≠ R₂ may be prepared by an analogous approach reacting naphthalene bisanhydride of formula (2) first with 1 equivalent of the amine R₁-NH₂ of formula (3) to afford the intermediate of formula (4). Subsequently the intermediate of formula (4) is reacted with 1 equivalent of the amine of formula (5) yielding the desired product of formula (1) in accordance with the following reaction scheme: 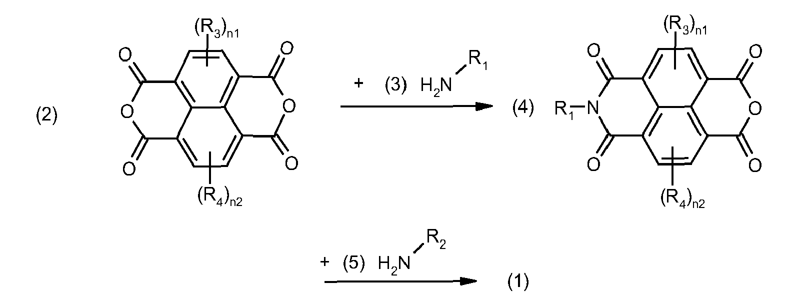 wherein
R₁, R₂, R₃, n1 and n2 are defined as in formula (1).

The preparation of derivatives of formula (1) may be accomplished in 2 steps or as a one-pot reaction.

The compounds of the formula (1) according to the present invention are particularly suitable as UV filters, i.e. for protecting ultraviolet-sensitive organic materials, in particular the skin and hair of humans and animals, from the harmful effects of UV radiation. These compounds are therefore suitable as sunscreens in cosmetic, pharmaceutical and veterinary medical preparations. These compounds can be used both in dissolved form and in the micronized state.

The UV absorbers according to the present invention are preferably used in the dissolved state (soluble organic filters, solubilized organic filters).

The cosmetic formulations or pharmaceutical compositions according to the present invention may additionally contain one or more than one further UV filter as listed in Table 2.

| Table 2: Suitable UV filter substances which can be additionally used with the UV absorbers according to the present invention (Abbreviations T: Table, R: row, Comp: compound, Ex: compound(s) of Patent Example, p: page; the generic scope of the UV absorbers is described in the left-hand column; specific compounds are indicated in the right-hand column) | |
|---|---|
| DE 10013318 | T 1 pp 8-9, all Examples pp 10-13, T 2 pp 13-14, all Examples p 14, Ex A, B, C, D, E, F pp 19-20 |
| DE 10206562 A1 | Ex 1-3 p 10, Ex 4-7 p 11, Ex 8-15 pp 12-14 |
| DE 10238144 A1 | Ex on p 3-5; |
| DE 10331804 | T 1 p 4, T 2 + 3 p 5 |
| DE 19704990 A1 | Ex 1-2 on pp 6-7; |
| EP 613 893 | Ex1-5+15,T1,pp6-8 |
| EP 0 998 900 A1 | Ex on pp 4-11 |
| EP 1 000 950 | Comp. In Table 1, pp 18-21 |
| EP 1 005 855 | T 3, p 13 |
| EP 1 008 586 | Ex 1-3, pp 13-15 |
| EP 1 008 593 | Ex 1-8, pp 4-5 |
| EP 1 027 883 | Compound VII, p 3 |
| EP 1 027 883 | Comp I-VI, p 3 |
| EP 1 028 120 | Ex 1-5, pp 5-13 |
| EP 1 059 082 | Ex 1; T 1, pp 9-11 |
| EP 1 060 734 | T 1-3, pp 11-14 |
| EP 1 064 922 | Compounds 1-34, pp 6-14 |
| EP 1 077 246 A2 | Ex 1-16 on pp 5-11; |
| EP 1 081 140 | Ex 1-9, pp 11-16 |
| EP 1 103 549 | Compounds 1-76, pp 39-51 |
| EP 1 108 712 | 4,5-Dimorph olino-3-hydroxypyridazine |
| EP 1 123 934 | T3,p10 |
| EP 1 129 695 | Ex 1-7, pp 13-14 |
| EP 1 167 359 | Ex 1 , p 11 and Ex 2, p 12 |
| EP 1 232 148 B1 | Ex 4-17 on pp 3-5; |
| EP 1 258 481 | Ex 1, pp 7,8 |
| EP 1 310 492 A1 | Ex 1-16 on pp 22-30 |
| EP 1 371 654 A1 | Ex on pp 5-7 |
| EP 1 380 583 A2 | Ex 1, p 6; |
| EP 1 423 351 A2 | Ex 1-16 on pp 31-37; |
| EP 1 423 371 A1 | T 1 on pp 4-8, Ex on p 9, Ex 1-9 on pp 36-42; |
| EP 1 454 896 A1 | Ex 1-5 on pp 10-13, Examples on pp 4-5; |
| EP 1 471 059 A1 | Ex 1-5 on pp 4-5; |
| EP 1484051 A2 | Formula III-VII on pp18-19, Ex 7-14 on pp 7-9, Ex 18-23 on pp 11-12, Ex 24-40 on pp 14-17; |
| EP 420 707 B1 | Ex 3, p 13 (CAS Reg. No 80142-49-0) |
| EP 503 338 | T 1, pp 9-10 |
| EP 517 103 | Ex 3,4,9,10 pp 6-7 |
| EP 517 104 | Ex 1, T 1, pp 4-5; Ex 8, T 2, pp 6-8 |
| EP 626 950 | all compounds |
| EP 669 323 | Ex 1-3, p 5 |
| EP 743 309 A1 | Ex 1-12 on pp 18-24; |
| EP 780 382 | Ex 1-11, pp 5-7 |
| EP 823 418 | Ex 1-4, pp 7-8 |
| EP 826 361 | T 1, pp 5-6 |
| EP 832 641 | Ex5+6p7;T2,p8 |
| EP 832 642 | Ex 22, T 3, pp 10-15; T 4, p 16 |
| EP 852 137 | T 2, pp 41-46 |
| EP 858 318 | T 1, p 6 |
| EP 863 145 | Ex 1-11, pp 12-18 |
| EP 878 469 A1 | T 1, pp 5-7; |
| EP 895 776 | Comp. In rows 48-58, p 3; R 25+33, p 5 |
| EP 911 020 | T 2, pp 11-12 |
| EP 916 335 | T 2-4, pp 19-41 |
| EP 924 246 | T2, p 9 |
| EP 933 376 | Ex 1-15, pp 10-21 |
| EP 944 624 | Ex 1+2, pp 13-15 |
| EP 945 125 | T 3 a+b, pp 14-15 |
| EP 95 097 | Ex 1, p 4 |
| EP 967 200 | Ex 2; T 3-5, pp 17-20 |
| EP 969 004 | Ex 5, T 1, pp 6-8 |
| FR 2842806 A1 | Ex I p 10, Ex II p 12 |
| FR 2861075 A1 | Ex 1-3 on pp 12-14; |
| FR 2862641 | Formula 3 on p4; Ex A-J on pp 7-9; |
| KR 2004025954 | all kojyl benzoate derivatives |
| JP 06135985 A2 | Formula 1 on p 2; Ex 1-8 on pp 7-8; |
| JP 2000319629 | CAS Reg Nos. 80142-49-0, 137215-83-9, 307947-82-6 |
| JP 2003081910 A | Ex on p 1; |
| JP 3686911 B2 | All benzylidene-gamma-butyrolactone derivatives |
| US 2003/0053966A1 | Ex on pp 3-6 |
| US 2004057912 A1 | Ex on p 7-9, Ex 1 on p 10; |
| US 2004057914 A1 | Ex on p 8-12, Ex 1 on p 12; |
| US 2004/0057911A1 | Formula I and II on p 1; formula III and IV on p3; Ex 1-3 on pp 5-6; |
| US 2004/0071640A1 | Ex 1-12 on pp 4-7; |
| US 2004/0091433A1 | Ex 1-6 on pp 14-16; |
| US 2004/0136931A1 | Ex 1-3 on p 7; |
| US 2004/0258636A1 | Ex 1-11 on pp 9-15; |
| US 2005/0019278A1 | Ex 1-9 on pp 6-8; |
| US 2005/0136012A1 | Formula 1 on p 2; |
| US 2005/0136014A1 | Formula a-c on p 2; Examples on p 3; |
| US 2005/0201957A1 | Formula 1 on p1; Ex A, B, C, D, E, F, G on pp 2-3; |
| US 2005/0249681A1 | all compounds on pp 2-3, Ex 1 on p 6; |
| US 5 635 343 | all compounds on pp 5-10 |
| US 5 332 568 | Ex 1, p 5, T 1+2, pp 6-8 |
| US 5 338 539 | Ex 1-9, pp 3+4 |
| US 5 346 691 | Ex 40, p 7; T 5, p 8 |
| US 5 801 244 | Ex 1-5, pp 6-7 |
| US 6613340 | Ex I, II pp 9-11, Examples on rows 28-53 p 6 |
| US 6 800 274 B2 | Formulas I-VI and IX-XII on pp 14-18; |
| US 6 890 520 B2 | Ex 1-10 on pp 6-9; |
| US 6926887 B2 | Ex A on pp5/6; Formulas I - VIII on pp 27-29; |
| US 6936735 B2 | Formula 1-2 on p 2; formula 3-4 on p 6; |
| WO 0149686 | Ex 1-5, pp 16-21 |
| WO 0168047 | Tables on pp 85-96 |
| WO 0181297 | Ex 1-3, pp 9-11 |
| WO 0191695 | Formula I on p 4, T on p 8 |
| WO 0202501 A1 | Ex la-c, p 5 |
| WO 02069926 A1 | Ex on p 9, Ex on pp 17-23 |
| WO 02072583 | T on pp 68-70 |
| WO 02080876 | Ex 1 on pp 7-9 |
| WO 0238537 | All compounds p 3, compounds on rows 1-10 p 4 |
| WO 03004557 A1 | Ex A1-A29 on pp 36-57; |
| WO 03007906 | Ex I-XXIII, pp 42-48 |
| WO 03086341 A2 | Formula 2-21, pp 4-6; |
| WO 03092643 A1 | T on pp 34-35, compounds listed on p 16 |
| WO 03097577 A1 | Ex on pp 6-8; Ex 1-3 on pp 15-18; |
| WO 03104183 A1 | Formula I-IV on p 1; Ex 1-5 on pp 27-28; |
| WO 04000256 A1 | Ex 1-10 on pp 18-24 |
| WO 04020398 A1 | Ex 1-3 on pp 14-17 |
| WO 04020398 A1 | Formulas I-VI on pp 21-24, Formula IX on p 25; |
| WO 05009938 A2 | Formula I on p 1; Ex 1-2 on pp 14-15; |
| WO 05065154 A2 | Formula a-c on pp 5-6; |
| WO 05080341 A1 | Formula 1 on p 3; Examples on pp 9-13; |
| WO 9217461 | Ex 1-22, pp 10-20 |
| WO 9220690 | Polymeric Comp in Examples 3-6 |
| WO 9301164 | T 1+2, pp 13-22 |
| WO 9714680 | Ex 1-3, p 10 |

The cosmetic or pharmaceutical preparations can be prepared by physically mixing the UV absorber(s) with the adjuvant using customary methods, for example by simply stirring together the individual components, especially by making use of the dissolution properties of already known cosmetic UV absorbers, like octyl methoxy cinnamate, salicylic acid isooctyl ester, etc. The UV absorber can be used, for example, without further treatment, or in the micronised state, or in the form of a powder.

Cosmetic or pharmaceutical preparations contain from 0.05-40% by weight, based on the total weight of the composition, of one UV absorber or UV absorber mixtures.

Preference is given to the use of mixing ratios of the UV absorber of formula (1) according to the present invention and optionally further light-protective agents (as described in Table 2) from 1:99 to 99:1, preferably from 5:95 to 95:5 and most preferably from 10:90 to 90:10, based on weight. Of special interest are mixing ratios of from 20:80 to 80:20, preferably from 40:60 to 60:40 and most preferably approximately 50:50. Such mixtures can be used, *inter alia,* to improve the solubility or to increase UV absorption.

The UV absorbers of formula (1) according to the present invention or combinations of UV filters are useful to protect skin, hair and/or natural or artificial hair color.

The cosmetic or pharmaceutical preparations may be, for example, creams, gels, lotions, alcoholic and aqueous/alcoholic solutions, emulsions, wax/fat compositions, stick preparations, powders or ointments. In addition to the above mentioned UV filters, the cosmetic or pharmaceutical preparations may contain further adjuvants as described below.

As water- and oil-containing emulsions (e.g. W/O, O/W, O/W/O and W/O/W emulsions or microemulsions) the preparations contain, for example, from 0.1 to 30 % by weight, preferably from 0.1 to 15 % by weight and especially from 0.5 to 10 % by weight, based on the total weight of the composition, of one or more UV absorbers, from 1 to 60 % by weight, especially from 5 to 50 % by weight and preferably from 10 to 35 % by weight, based on the total weight of the composition, of at least one oil component, from 0 to 30 % by weight, especially from 1 to 30 % by weight und preferably from 4 to 20 % by weight, based on the total weight of the composition, of at least one emulsifier, from 10 to 90 % by weight, especially from 30 to 90 % by weight, based on the total weight of the composition, of water, and from 0 to 88.9 % by weight, especially from 1 to 50 % by weight, of further cosmetically acceptable adjuvants.

The cosmetic or pharmaceutical compositions/preparations according to the invention may also contain one or one more additional compounds like fatty alcoholsEsters of fatty acids, natural or synthetic triglycerides including glyceryl esters and derivatives, pearlescent waxes, hydrocarbon oils, silicones or siloxanes (organosubstituted polysiloxanes), fluorinated or perfluorinated oils, emulsifiers, super-fatting agents, surfactants, consistency regulators/thickeners and rheology modifiers, polymers, biogenic active ingredients, deodorising active ingredients, anti-dandruff agents, antioxidants, hydrotropic agents, preservatives and bacteria-inhibiting agents, perfume oils, colourants, polymeric beads or hollow spheres as SPF enhancers.

### Cosmetic or pharmaceutical preparations

Cosmetic or pharmaceutical formulations are contained in a wide variety of cosmetic preparations. There come into consideration, for example, especially the following preparations:
- skin-care preparations, e.g. skin-washing and cleansing preparations in the form of tablet-form or liquid soaps, soapless detergents or washing pastes,
- bath preparations, e.g. liquid (foam baths, milks, shower preparations) or solid bath preparations, e.g. bath cubes and bath salts;
- skin-care preparations, e.g. skin emulsions, multi-emulsions or skin oils;
- cosmetic personal care preparations, e.g. facial make-up in the form of day creams or powder creams, face powder (loose or pressed), rouge or cream make-up, eye-care preparations, e.g. eyeshadow preparations, mascara, eyeliner, eye creams or eye-fix creams; lip-care preparations, e.g. lipsticks, lip gloss, lip contour pencils, nail-care preparations, such as nail varnish, nail varnish removers, nail hardeners or cuticle removers;
- foot-care preparations, e.g. foot baths, foot powders, foot creams or foot balsams, special deodorants and antiperspirants or callus-removing preparations;
- light-protective preparations, such as sun milks, lotions, creams or oils, sunblocks or tropicals, pre-tanning preparations or after-sun preparations;
- skin-tanning preparations, e.g. self-tanning creams;
- depigmenting preparations, e.g. preparations for bleaching the skin or skin-lightening preparations;
- insect-repellents, e.g. insect-repellent oils, lotions, sprays or sticks;
- deodorants, such as deodorant sprays, pump-action sprays, deodorant gels, sticks or roll-ons;
- antiperspirants, e.g. antiperspirant sticks, creams or roll-ons;
- preparations for cleansing and caring for blemished skin, e.g. synthetic detergents (solid or liquid), peeling or scrub preparations or peeling masks;
- hair-removal preparations in chemical form (depilation), e.g. hair-removing powders, liquid hair-removing preparations, cream- or paste-form hair-removing preparations, hair-removing preparations in gel form or aerosol foams;
- shaving preparations, e.g. shaving soap, foaming shaving creams, non-foaming shaving creams, foams and gels, preshave preparations for dry shaving, aftershaves or aftershave lotions;
- fragrance preparations, e.g. fragrances (eau de Cologne, eau de toilette, eau de parfum, parfum de toilette, perfume), perfume oils or perfume creams;
- cosmetic hair-treatment preparations, e.g. hair-washing preparations in the form of shampoos and conditioners, hair-care preparations, e.g. pretreatment preparations, hair tonics, styling creams, styling gels, pomades, hair rinses, treatment packs, intensive hair treatments, hair-structuring preparations, e.g. hair-waving preparations for permanent waves (hot wave, mild wave, cold wave), hair-straightening preparations, liquid hair-setting preparations, hair foams, hairsprays, bleaching preparations, e.g. hydrogen peroxide solutions, lightening shampoos, bleaching creams, bleaching powders, bleaching pastes or oils, temporary, semi-permanent or permanent hair colourants, preparations containing self-oxidising dyes, or natural hair colourants, such as henna or camomile.

### Presentation forms

The final formulations listed may exist in a wide variety of presentation forms, for example:
- in the form of liquid preparations as a W/O, O/W, O/W/O, W/O/W or PIT emulsion and all kinds of microemulsions,
- in the form of a gel,
- in the form of an oil, a cream, milk or lotion,
- in the form of a powder, a lacquer, a tablet or make-up,
- in the form of a stick,
- in the form of a spray (spray with propellent gas or pump-action spray) or an aerosol,
- in the form of a foam, or
- in the form of a paste.

Of special importance as cosmetic preparations for the skin are light-protective preparations, such as sun milks, lotions, creams, oils, sunblocks or tropicals, pretanning preparations or after-sun preparations, also skin-tanning preparations, for example self-tanning creams. Of particular interest are sun protection creams, sun protection lotions, sun protection milk and sun protection preparations in the form of a spray.

Of special importance as cosmetic preparations for the hair are the above-mentioned preparations for hair treatment, especially hair-washing preparations in the form of shampoos, hair conditioners, hair-care preparations, e.g. pretreatment preparations, hair tonics, styling creams, styling gels, pomades, hair rinses, treatment packs, intensive hair treatments, hair-straightening preparations, liquid hair-setting preparations, hair foams and hairsprays. Of special interest are hair-washing preparations in the form of shampoos.

A shampoo has, for example, the following composition: from 0.01 to 5 % by weight of a UV absorber according to the invention, 12.0 % by weight of sodium laureth-2-sulfate, 4.0 % by weight of cocamidopropyl betaine, 3.0 % by weight of sodium chloride, and water ad 100%.

The cosmetic preparation according to the invention is distinguished by excellent protection of human skin against the damaging effect of sunlight.

### Examples

### A. Preparation Examples

### Example A1: Preparation of the compound of formula

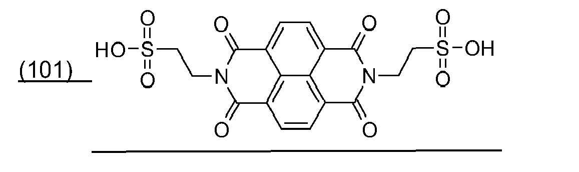

Naphthalenetetracarboxylic bisanhydride (1.41g, 5mmol) and taurine (1.26g, 10mmol) are dispersed in N,N-dimethylformamide (65ml).
The mixture is heated at 150°C for 19h and cooled to room temperature.
A beige product precipitates which is isolated by filtration and dried in vacuo yielding the desired product (2.11g) in yields of 87 %.

Solubility in water: > 30 G/G%.

### UV spectrum of the compound of formula (101) (measured in H₂O):

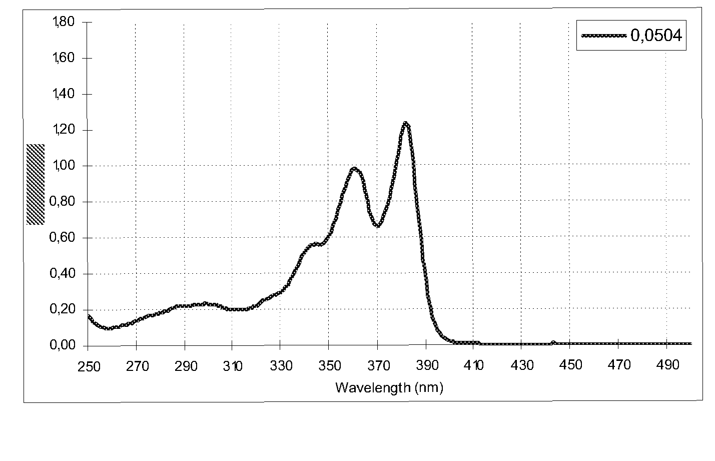

## Claims

1. Use of the compounds of formula 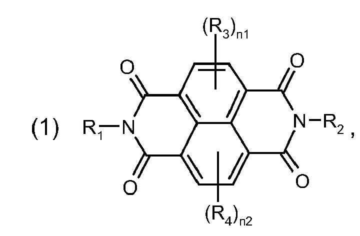 wherein
R₁ and R₂ independently of each other are hydrogen; C₁-C₂₈alkyl, C₂-C₂₈alkenyl, C₂-C₂₂alkinyl, C₃-C₁₂cycloalkyl, C₃-C₁₂cycloalkenyl, C₇-C₂₈aralkyl, C₁-C₂₀heteroalkyl, C₃-C₁₂cycloheteroalkyl, C₅-C₁₆heteroaralkyl, which are substituted by one or more groups of Z and/or interrupted by -O-; -NR₆-; or CO-R₅;
with the proviso that R₁ and R₂ are not simultaneously hydrogen;
Z is COO(M)ₘ *-SO₃(M)ₘ; or -SO₂NR₆R₇;
M is hydrogen; or a cation;
m is 1; 0.5; or ¹/₃;
R₃ and R₄, independently from each other are C₁-C₂₈alkyl; C₂-C₂₈ alkenyl; C₂-C₂₈alkinyl; C₃-C₁₂cycloalkyl; C₃-C₁₂cycloalkenyl; C₇-C₂₀aralkyl; C₂-C₂₈alkoxy; or OH;
R₅, R₆ and R₇ independently from each other are hydrogen; C₁-C₂₈alkyl; hydroxyl-C₁-C₂₈alkyl; C₂-C₂₈alkenyl; C₂-C₂₈alkinyl; C₃-C₁₂cycloalkyl; C₃-C₁₂cycloalkenyl; C₇-C₂₀aralkyl; C₂-C₂₈ alkoxy; or OH; and
n1 and n2, independently from each other are0; 1; or 2;
for protecting human and animal hair and skin from UV radiation.

2. Use according to claim 1, wherein
R₁ and R₂ independently of each other are hydrogen; or C₁-C₂₈alkyl, which are substituted by one or more groups of Z and/or interrupted by -O- or -NR₆-; wherein
Z and R₆ are defined as in claim 1.

3. Use according to claim 2, wherein
R₁ and R₂ independently of each other are C₁-C₅alkyl.

4. Use according to any of the proceeding claims, wherein
R₁ and R₂ have the same meaning.

5. Use according to any of the proceeding claims, wherein
M is a metal cation selected from sodium, potassium, calcium, magnesium and lithium; or an organic cation selected from ammonium and triethanolammonium.

6. Use according to any of the proceeding claims, wherein
n1 and n2 are 0.

7. Use according to any of the proceeding claims, wherein
Z is *-SO₃H; *-SO₂NH₂ or *-COOM; wherein
M is hydrogen; or an alkali metal cation.

8. Use according to any of the proceeding claims, wherein the compounds correspond to formula 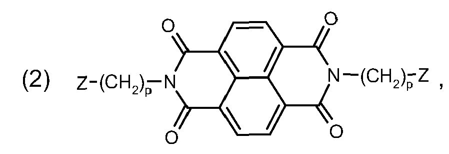 wherein
Z is *-SO₃H; *-SO₂NH₂ or *-COOM;
M is hydrogen; or an alkali metal cation; and
p is a number from 1 to 5; and
wherein the (CH₂)-chains may be substituted by hydroxyl, -NR₆R₇ and/or interrupted by -O-; and
R₆ and R₇ are defined as in claim 1.

9. Process for the preparation of compounds of formula (1), wherein R₁ = R₂, which process comprises reacting the compound of formula (2) with 2 moles of formula (3) according to the following reaction scheme: 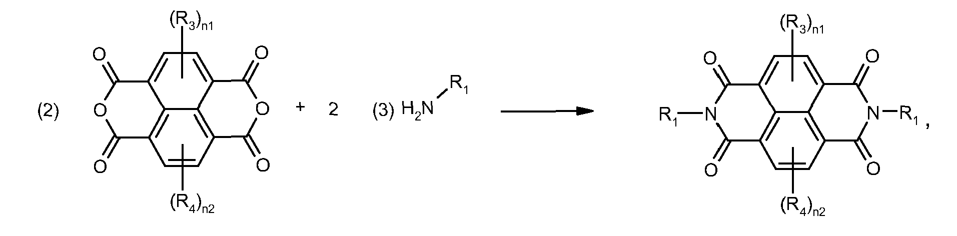 wherein
R_{1'} R₃, n1 and n2 are defined as in claim 1.

10. Process for the preparation of compounds of formula (1), wherein R₁ ≠ R₂, which process comprises reacting the compound of formula (2) with 1 mol of the amine of formula (3) to obtain the intermediate of formula (4) and reacting the intermediate with the amine of formula (5) according to the following reaction scheme: 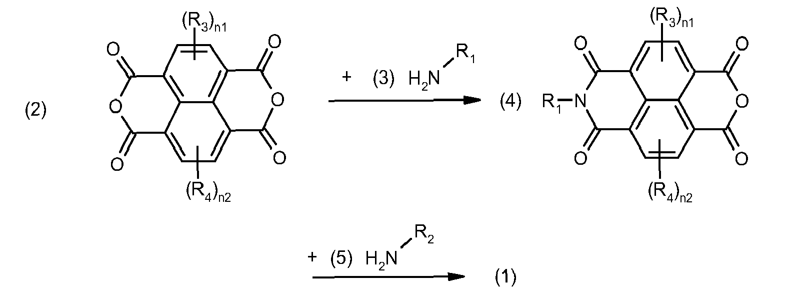 wherein
R₁, R₂, R₃, n1 and n2 are defined as in claim 1.

11. Cosmetic composition comprising a compound of formula (1) according to claim 1 and at least one cosmetic acceptable carrier.

12. Compounds of formula 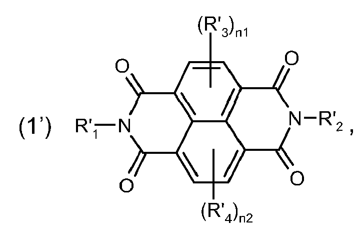 wherein
R'₁ and R'₂ independently of each other are hydrogen; C₁-C₂₈alkyl, C₂-C₂₈alkenyl, C₂-C₂₂alkinyl, C₃-C₁₂cycloalkyl, C₃-C₁₂cycloalkenyl, C₇-C₂₈aralkyl, C₁-C₂₀heteroalkyl, C₃-C₁₂cycloheteroalkyl, C₅-C₁₆heteroaralkyl, which are substituted by one or more groups of Z' and/or interrupted by -O- or -NR'₆-;
with the proviso that R'₁ and R'₂ are not simultaneously hydrogen;
Z' is *-SO₃(M')ₘ; or *-SO₂NR'₆R'7₈;
M' is hydrogen; or a cation;
m is 1; 0.5; or ¹/₃;
R'₃ and R'₄ independently from each other are C₁-C₂₈alkyl; C₂-C₂₈ alkenyl; C₂-C₂₈alkinyl; C₃-C₁₂cycloalkyl; C₃-C₁₂cycloalkenyl; C₇-C₂₀aralkyl; C₂-C₂₈alkoxy; or OH;
R'₅, R'₆ and R'₇ independently from each other are hydrogen; C₁-C₅alkyl; or hydroxy-C₁-C₅alkyl; and
n1 and n2 independently from each other are 0; 1; or 2.
